# EUROPEAN PATENT SPECIFICATION

(11) **EP 2 781 238 B1**
(45) Date of publication and mention of the grant of the patent: **08.11.2017**
(21) Application number: 12849544.7
(22) Date of filing: 27.02.2012
(51) Int. Cl.: A63B 21/02

(54) **MUSCLE-BUILDING IMPLEMENT AND METHOD OF CONTROLLING SAME**
MUSKELAUFBAUENDE VORRICHTUNG UND STEUERUNGSVERFAHREN DAFÜR
ACCESSOIRE DE MUSCULATION ET SON PROCÉDÉ DE COMMANDE

(30) Priority: 17.11.2011 JP 2011251493
(43) Date of publication of application: 24.09.2014
(73) Proprietor: Sato, Yoshiaki, Tokyo 183-0016 (JP)
(72) Inventor: Sato, Yoshiaki, Tokyo 183-0016 (JP)
(74) Representative: Gill Jennings & Every LLP
(86) International application number: PCT/JP2012/054756
(87) International publication number: WO 2013/073205

(56) References cited:
- EP-A1- 2 105 169
- WO-A1-2009/128561
- JP-A- H1 085 362
- JP-A- H07 144 027
- JP-A- 2005 006 921
- JP-A- 2005 058 544
- JP-A- 2008 099 842
- US-A1- 2006 201 522

## Description

### Technical Field

The present invention relates to a muscle building tool and a control method thereof.

### Background Art

A pressure muscle strength training method capable of efficiently building a muscle by applying a load by blood flow restriction to the muscle is currently proposed and put to practical use (for example, see Patent document 1). A muscle building tool that applies a pressure to a muscle by tightening a belt around a specific part of a user's four limbs (arms and legs) is used in such a muscle strength training method.

To implement the pressure muscle strength training method using the muscle building tool, it is essential to apply an accurate pressure to a specific part of muscles to be built, at a position accurate to a certain extent. Accordingly, a muscle building tool including a pressure measurement device for obtaining, as a gas pressure in a pressure receiving bag, a pressure applied to a muscle by a belt, transmitting the pressure to a pressure sensitive element by transmission means, and measuring the transmitted pressure by the pressure sensitive element is proposed in recent years (for example, see Patent document 2).

### Prior art document(s)

- Patent document 1:: Japanese Patent No. 2670421
- Patent document 2:: Japanese Patent No. 2796277
- Patent document 3:: US Patent No. 2006/201522 A1
discloses a method for lowering blood glucose levels and a method for treating and preventing diabetes by restricting blood flow to a muscle of a limb, according to the preamble of claim 1.

### Summary of the Invention

### Problems to be solved by the Invention

However, in the above-mentioned conventional muscle building tool including the pressure measurement device, the pressure receiving bag whose internal gas pressure varies according to the applied pressure and a measurement tank in which the pressure sensitive element and the like are housed need to be connected for communication via a tube (hollow elastic tube). This makes the whole tool large and bulky, and raises a possibility that training is hindered by the tube in particular.

Besides, in the above-mentioned conventional muscle building tool, the pressure receiving bag is connected, via the tube, to the measurement tank placed at an end of the belt, and the tube is limited in length. Thus, there is a constraint that requires the pressure receiving bag to be placed near the end of the belt, too. If the pressure receiving bag is placed near the end of the belt, a gap might be formed between the pressure receiving bag and the user's arm (or leg). In such a case, a problem that the pressure applied to the muscle cannot be accurately detected by the pressure receiving bag arises.

The present invention has been made in view of these circumstances, and has an object of providing a muscle building tool capable of accurately measuring a pressure applied to a muscle by a belt with no need for a pressure receiving bag or a tube.

### Means for solving the Problems

To achieve the object stated above, according to the present invention a muscle building tool as claimed in claim 1 is provided.

With such a structure, in the case where the tension acting on the belt when wrapping the belt exceeds the predetermined threshold, the pressure measurement device can be automatically operated to measure the pressure. This eliminates the need to provide and manipulate an additional switch or the like for operating the pressure measurement device, and enables the measurement of the pressure to be started immediately after the belt is wrapped around, for example, the user's upper arm. Since the muscle building tool does not need a pressure receiving bag or a tube unlike the conventional muscle building tool, the tool as a whole is compact and easy to use during training.

In the muscle building tool, the pressure measurement device including: a pressure sensitive element that is placed in a longitudinally substantially middle area of a surface of the belt facing the muscle, and outputs an electrical signal corresponding to the pressure applied to the muscle; and a calculation unit that calculates the pressure based on the electrical signal output from the pressure sensitive element may be used.

With such a structure, the pressure sensitive element in the pressure measurement device is placed in the longitudinally substantially middle area of the belt (position away from each end of the belt). This ensures that, for example when the belt is wrapped around the user's upper arm and the pressure is applied while holding an end of the belt, the pressure sensitive element at the position away from each end of the belt is closely attached to the upper arm. Hence, a situation where the pressure cannot be accurately measured due to a gap formed between the pressure sensitive element and the human body can be avoided, which contributes to improved pressure measurement accuracy.

Moreover, in the muscle building tool, it is preferable that a plurality of pressure sensitive elements are arranged at predetermined intervals in the longitudinally substantially middle area of the internal surface of the belt. In such a case, the calculation unit in the pressure measurement device may calculate a mean value of the pressure based on a plurality of electrical signals output from the plurality of pressure sensitive elements.

With such a structure, it is possible to arrange the plurality of pressure sensitive elements and calculate the mean value of the pressure based on the plurality of electrical signals output from the plurality of pressure sensitive elements. This reduces the possibility of any measurement error as compared with the case of measuring the pressure using a single pressure sensitive element, so that the pressure measurement accuracy can be further improved.

Moreover, in the muscle building tool, the pressure measurement device configured to automatically stop the operation when, after the pressure measurement device is activated, the tension measured by the tension measurement device falls below or equal to the predetermined threshold may be used.

With such a structure, in the case where the tension acting on the belt falls below or equal to the predetermined threshold, the operation of the pressure measurement device can be automatically stopped. This eliminates the need to provide and manipulate an additional switch or the like for stopping the pressure measurement device, and enables the measurement of the pressure to be stopped immediately after the belt is removed. In addition, in the case of using a pressure measurement device that operates with supplied power, this automatic stop function can prevent wasting power.

Moreover, the muscle building tool may further include: an information storage medium configured to store various information; and a storage control unit that stores the pressure calculated by the calculation unit in the pressure measurement device, in the information storage medium.

With such a structure, the pressure (actual pressure) calculated by the calculation unit in the pressure measurement device can be stored in the information storage medium. Therefore, the level of the actual pressure can be confirmed subsequently.

Moreover, in the muscle building tool, the storage control unit that stores a maximum value of the pressure calculated by the calculation unit during the operation of the pressure measurement device in the information storage medium may be used.

With such a structure, the maximum value of the actual pressure during the operation of the pressure measurement device can be confirmed subsequently.

Moreover, in the muscle building tool, the storage control unit that, in the case where the pressure calculated by the calculation unit in the pressure measurement device exceeds a proper pressure and a state in which the pressure calculated by the calculation unit exceeds the proper pressure continues for a fixed period, stores information that the state in which the pressure calculated by the calculation unit exceeds the proper pressure continues for the fixed period in the information storage medium may be used.

With such a structure, in the case where the state in which the actual pressure exceeds the proper pressure continues for the fixed period, the information that the state in which the actual pressure exceeds the proper pressure continues for the fixed period can be stored in the information storage medium. This enables the user to subsequently confirm the fact that the actual pressure exceeding the proper pressure is continuously applied for the fixed period.

Moreover, the muscle building tool may further include: a display device for displaying various information; and a display control unit that displays various information including the pressure calculated by the calculation unit in the pressure measurement device, on the display device. Further, it is preferable that a folding portion is provided at one end of the belt, the folding portion being used to fold the belt back by passing an other end of the belt through the folding portion when wrapping the belt, and that the display device is placed near the folding portion.

With such a structure, the display device is placed near the folding portion, so that the user can easily view information displayed on the display device. For example, when the belt is wrapped around the user's upper arm and the pressure is applied to the muscle while folding the belt back by passing, through the folding portion provided at one end of the belt, the other end of the belt, the user can easily view information such as the pressure because the display device is placed near the folding portion (placed at the position where the viewing by the user is easiest).

Moreover, in the muscle building tool, the display control unit that displays both the pressure calculated by the calculation unit in the pressure measurement device and a predetermined proper pressure on the display device may be used.

With such a structure, both the actual pressure and the proper pressure can be displayed on the display device. This enables the user to easily compare the actual pressure and the proper pressure, and adjust the pressure so that the actual pressure matches the proper pressure.

Moreover, in the muscle building tool, the display control unit that displays predetermined warning information on the display device in the case where the pressure calculated by the calculation unit in the pressure measurement device exceeds the proper pressure and a state in which the pressure calculated by the calculation unit exceeds the proper pressure continues for a fixed period may be used.

With such a structure, in the case where the state in which the actual pressure exceeds the proper pressure continues for the fixed period, the predetermined warning information can be displayed on the display device to inform the user.

Moreover, the muscle building tool may further include: a sound output device for outputting a sound; and a sound control unit that outputs a predetermined warning sound from the sound output device, in the case where the pressure calculated by the calculation unit in the pressure measurement device exceeds a predetermined proper pressure and a state in which the pressure calculated by the calculation unit exceeds the proper pressure continues for a fixed period.

With such a structure, in the case where the state in which the actual pressure exceeds the proper pressure continues for the fixed period, the predetermined warning sound can be output from the sound output device to inform the user.

Moreover, in the muscle building tool, the pressure measurement device that includes an activation number counting unit that counts the number of operations of the pressure measurement device may be used, and the muscle building tool may include a sound control unit that outputs a predetermined warning sound from the sound output device in the case where the number of operations counted by the activation number counting unit exceeds a predetermined number corresponding to an effective period of a predetermined proper pressure.

With such a structure, in the case where the number of uses of the muscle building tool (the number of operations of the pressure measurement device) exceeds the predetermined number, the predetermined warning sound can be output from the sound output device to inform the user on the assumption that the effective period of the proper pressure set on the user basis has elapsed. This enables the user to know that the effective period of the set proper pressure has elapsed, and set a new proper pressure.

Moreover, in the muscle building tool, the sound control unit that outputs a predetermined warning sound from the sound output device in the case where the number of operations counted by the activation number counting unit in the pressure measurement device exceeds a predetermined number corresponding to a durable period of the muscle building tool may be used.

With such a structure, in the case where the number of uses of the muscle building tool (the number of operations of the pressure measurement device) exceeds the predetermined number, the predetermined warning sound can be output from the sound output device to inform the user on the assumption that the durable period of the muscle building tool has elapsed. This enables the user to know that the durable period of the muscle building tool has elapsed and it is time to purchase a new muscle building tool.

Moreover, in the muscle building tool, the pressure measurement device may include a function stop unit that stops a function of the pressure measurement device in the case where the number of operations counted by the activation number counting unit exceeds the predetermined number corresponding to the durable period of the muscle building tool.

With such a structure, in the case where the number of uses of the muscle building tool (the number of operations of the pressure measurement device) exceeds the predetermined number, the function of the pressure measurement device can be stopped on the assumption that the durable period of the muscle building tool has elapsed. The user can therefore be prevented from mistakenly using the muscle building tool whose durable period has elapsed.

Moreover, a control method according to the present invention is a control method of a muscle building tool that includes: a belt that is wrapped around at least one of a user's four limbs and applies a predetermined pressure to a muscle of the limb; a shape maintenance member that maintains a loop shape of the belt in a state of applying the pressure to the muscle; a tension measurement device that measures a tension acting on the belt; and a pressure measurement device that measures the pressure applied to the muscle, the control method including an operation start step of automatically starting an operation of the pressure measurement device when the tension measured by the tension measurement device exceeds a predetermined threshold.

With such a method, in the case where the tension acting on the belt when wrapping the belt exceeds the predetermined threshold, the pressure measurement device can be automatically operated to measure the pressure. This eliminates the need to provide and manipulate an additional switch or the like for operating the pressure measurement device, and enables the measurement of the pressure to be started immediately after the belt is wrapped around, for example, the user's upper arm.

The control method may include an operation stop step of automatically stopping the operation of the pressure measurement device when, after the operation start step, the tension measured by the tension measurement device falls below or equal to the predetermined threshold.

With such a method, in the case where the tension acting on the belt falls below or equal to the predetermined threshold, the operation of the pressure measurement device can be automatically stopped. This eliminates the need to provide and manipulate an additional switch or the like for stopping the pressure measurement device, and enables the measurement of the pressure to be stopped immediately after the belt is removed.

Moreover, the control method may include an information storage step of storing the pressure measured by the pressure measurement device, in an information storage medium.

With such a method, the pressure (actual pressure) measured by the pressure measurement device can be stored in the information storage medium. Therefore, the level of the actual pressure can be confirmed subsequently.

Moreover, the control method may include an information display step of displaying both the pressure measured by the pressure measurement device and a predetermined proper pressure, on a display device.

With such a method, both the pressure (actual pressure) measured by the pressure measurement device and the proper pressure can be displayed on the display device. This enables the user to easily compare the actual pressure and the proper pressure, and adjust the pressure so that the actual pressure matches the proper pressure.

Moreover, in the information display step in the control method, predetermined warning information may be displayed on the display device in the case where the pressure measured by the pressure measurement device exceeds the proper pressure and a state in which the pressure measured by the pressure measurement device exceeds the proper pressure continues for a fixed period.

With such a method, in the case where the state in which the actual pressure exceeds the proper pressure continues for the fixed period, the predetermined warning information can be displayed on the display device to inform the user.

Moreover, the control method may include a proper pressure excess warning sound output step of outputting a predetermined warning sound from a sound output device, in the case where the pressure measured by the pressure measurement device exceeds a predetermined proper pressure and a state in which the pressure measured by the pressure measurement device exceeds the proper pressure continues for a fixed period.

With such a method, in the case where the state in which the actual pressure exceeds the proper pressure continues for the fixed period, the predetermined warning sound can be output from the sound output device to inform the user.

Moreover, the control method may include: an operation number counting step of counting the number of operations of the pressure measurement device; and an effective period excess warning sound output step of outputting a predetermined warning sound from a sound output device, in the case where the number of operations counted in the operation number counting step exceeds a predetermined number corresponding to an effective period of a predetermined proper pressure.

With such a method, in the case where the number of uses of the muscle building tool (the number of operations of the pressure measurement device) exceeds the predetermined number, the predetermined warning sound can be output from the sound output device to inform the user on the assumption that the effective period of the proper pressure set on the user basis has elapsed. This enables the user to know that the effective period of the set proper pressure has elapsed, and set a new proper pressure.

Moreover, the control method may include a durable period excess warning sound output step of outputting a predetermined warning sound from a sound output device, in the case where the number of operations counted in the operation number counting step exceeds a predetermined number corresponding to a durable period of the muscle building tool.

With such a method, in the case where the number of uses of the muscle building tool (the number of operations of the pressure measurement device) exceeds the predetermined number, the predetermined warning sound can be output from the sound output device to inform the user on the assumption that the durable period of the muscle building tool has elapsed. This enables the user to know that the durable period of the muscle building tool has elapsed and it is time to purchase a new muscle building tool.

Moreover, the control method may include a function stop step of stopping a function of the pressure measurement device, in the case where the number of operations counted in the operation number counting step exceeds a predetermined number corresponding to a durable period of the muscle building tool.

With such a method, in the case where the number of uses of the muscle building tool (the number of operations of the pressure measurement device) exceeds the predetermined number, the function of the pressure measurement device can be stopped on the assumption that the durable period of the muscle building tool has elapsed. The user can therefore be prevented from mistakenly using the muscle building tool whose durable period has elapsed.

### Advantageous effect of the Invention

According to the present invention, it is possible to provide a muscle building tool capable of accurately measuring a pressure applied to a muscle by a belt with no need for a pressure receiving bag or a tube.

### Brief description of the Drawings

FIG. 1 is a view showing an internal surface (surface closely attached to the human body) of a muscle building tool according to an embodiment of the present invention.
FIG. 2 is a view showing an external surface of the muscle building tool shown in FIG. 1.
FIG. 3 is a side view of the muscle building tool shown in FIG. 1.
FIG. 4 is a block diagram for describing a functional structure of an information processing device in the muscle building tool shown in FIG. 1.
FIG. 5 is a flowchart for describing a use method of the muscle building tool shown in FIG. 1.
FIG. 6 is a view showing an internal surface (surface facing the muscle) of a muscle building tool according to another embodiment of the present invention.
FIG. 7 is a view showing an external surface (surface exposed to the outside) of the muscle building tool shown in FIG. 6.
FIG. 8 is a side view of the muscle building tool shown in FIG. 6.

### Mode for carrying out the Invention

The following describes an embodiment of the present invention with reference to drawings.

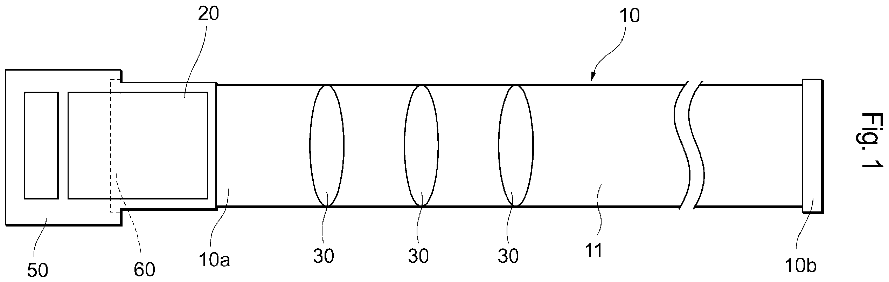

A structure of a muscle building tool 1 according to the embodiment of the present invention is described first, with reference to FIGS. 1 to 4.

The muscle building tool 1 according to this embodiment is used for "pressure muscle strength training" that builds, by applying a predetermined pressure to at least one of a user's four limbs to restrict blood flow, a muscle (or muscles) of the limb. The muscle building tool 1 includes: a belt 10; and an information processing device 20 shaped as a thin housing and attached to one end 10a of the belt 10, as shown in FIGS. 1 to 3.

The belt 10 is a band-shaped member that is wrapped around at least one of the user's four limbs and applies the pressure to the muscle, and is made of an elastic material as an example. A length and a width of the belt 10 can be appropriately set according to the user's physical constitution and the part around which the belt 10 is wrapped.

A plurality of pressure sensitive elements 30 arranged at predetermined intervals are attached to a longitudinally substantially middle area of an internal surface (surface closely attached to the human body) 11 of the belt 10, as shown in FIG. 1. Each pressure sensitive element 30 outputs an electrical signal corresponding to the pressure applied to the muscle, and constitutes a pressure measurement device (pressure sensor) in the present invention. The electrical signal output from each pressure sensitive element 30 is sent to the information processing device 20 via an electrical wire (not shown), and used for pressure calculation.

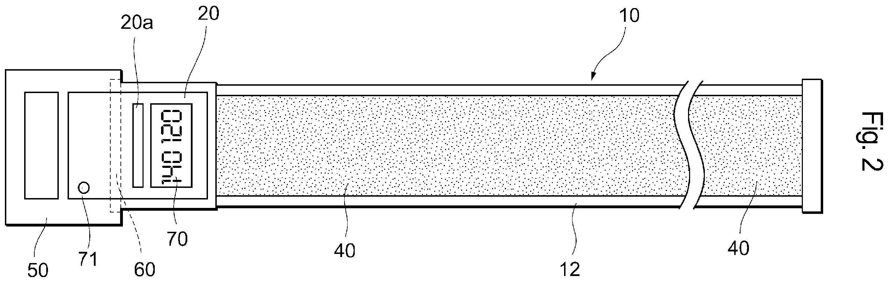

A hook-and-loop fastener 40 is provided on an external surface (surface exposed to the outside) 12 of the belt 10, as shown in FIG. 2. The hook-and-loop fastener 40 maintains a loop shape of the belt 10 in a state of applying the pressure to the muscle, and functions as a shape maintenance member in the present invention. A position at which the hook-and-loop fastener 40 is provided can be appropriately set according to the length of the belt 10 and the like. The hook-and-loop fastener 40 may be provided on the internal surface 11 of the belt 10.

A buckle 50 is attached to the end 10a of the belt 10 via the information processing device 20. The buckle 50 is used to fold the belt 10 back by passing the other end 10b of the belt through the buckle 50 when wrapping the belt 10, and functions as a folding portion in the present invention. A tension sensor 60 is provided at a connecting portion between the information processing device 20 and the buckle 50. The tension sensor 60 measures a tension acting on the belt 10 when wrapping the belt 10 around the user's limb, and functions as a tension measurement device in the present invention. Information about the tension measured by the tension sensor 60 is used for controlling ON/OFF of the information processing device 20.

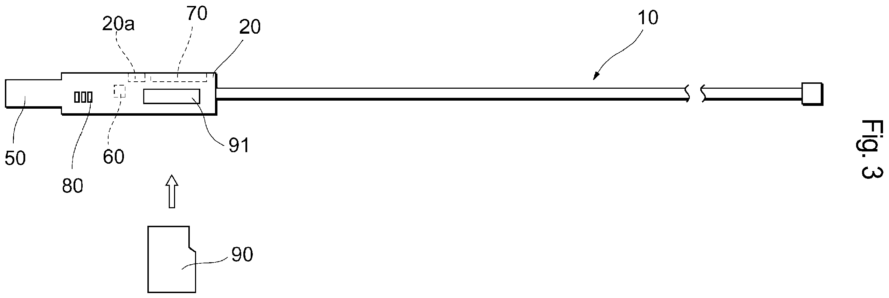

The information processing device 20 is a small computer system including a CPU for performing various processes and a memory in which control programs are recorded, and performs pressure calculation and various controls. The information processing device 20 includes: a digital display device 70; a sound output unit 80 for outputting various sounds; and a memory installation unit 91 for installing a memory card 90, as shown in FIGS. 2 and 3. In addition, a solar battery 20a is provided on an external surface (surface exposed to the outside) of the information processing device 20. The information processing device 20 is driven with power generated from the solar battery 20a upon receiving light.

The digital display device 70 is placed on the external surface of the information processing device 20, and displays various information such as the measured pressure. A warning display device 71 for displaying predetermined warning information is provided on the external surface of the information processing device 20. The digital display device 70 and the warning display device 71 are placed near the buckle 50, enabling the user using the muscle building tool 1 to easily view the display.

The sound output unit 80 and the memory installation unit 91 are placed on a side surface of the information processing device 20, as shown in FIG. 3. The memory card 90 corresponds to an information storage medium in the present invention, and is removable from the information processing device 20. In this embodiment, a pressure (proper pressure) suitable to the user is input to the memory card 90 by a pressure muscle strength trainer beforehand.

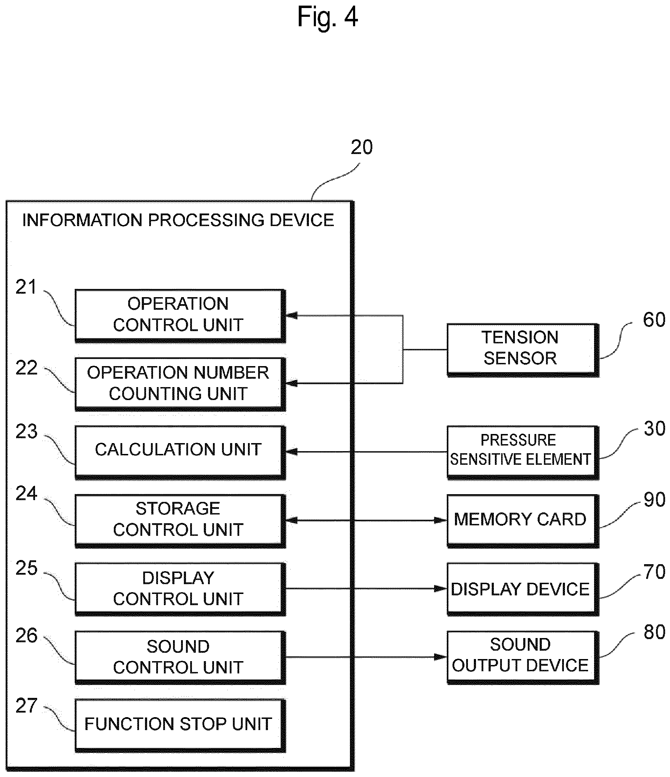

The following describes a functional structure of the information processing device 20. The information processing device 20 includes an operation control unit 21, an operation number counting unit 22, a calculation unit 23, a storage control unit 24, a display control unit 25, a sound control unit 26, and a function stop unit 27, as shown in FIG. 4.

The operation control unit 21 in the information processing device 20 functions to monitor the tension measured by the tension sensor 60 and, when the tension exceeds a predetermined threshold (e.g. 15 mmHg), automatically start the operation of the information processing device 20. The operation control unit 21 also functions to, when the tension measured by the tension sensor 60 falls below or equal to the predetermined threshold during the operation of the information processing device 20, automatically stop the operation of the information processing device 20.

The operation number counting unit 22 in the information processing device 20 functions to count the number of times the information processing device 20 automatically starts the operation by the operation control unit 21. The operation number counting unit 22 in this embodiment has a counting correction function of not counting the operation in the case where a period from when the information processing device 20 automatically starts the operation to when the information processing device 20 automatically stops the operation is below a predetermined period. A plurality of pulling operations upon wearing the belt are thus kept from being counted.

The calculation unit 23 in the information processing device 20 calculates the pressure applied to the muscle based on the electrical signal output from each pressure sensitive element 30, and constitutes the pressure measurement device (pressure sensor) in the present invention together with the pressure sensitive element 30. The calculation unit 23 in this embodiment calculates a plurality of pressures based on a plurality of electrical signals output from the plurality of pressure sensitive elements 30, and then calculates a mean value of the plurality of pressures.

The storage control unit 24 in the information processing device 20 functions to write (store) the pressure calculated by the calculation unit 23 to the memory card 90 installed in the memory installation unit 91. The storage control unit 24 in this embodiment writes a maximum value of the pressure calculated by the calculation unit 23 during the operation of the information processing device 20, to the memory card 90. Moreover, in the case where a state in which the pressure calculated by the calculation unit 23 exceeds the proper pressure continues for a fixed period (e.g. 10 seconds), the storage control unit 24 writes the information that the state in which the pressure calculated by the calculation unit 23 exceeds the proper pressure continues for the fixed period, to the memory card 90.

The display control unit 25 in the information processing device 20 functions to display various information including the pressure calculated by the calculation unit 23, on the digital display device 70. The display control unit 25 in this embodiment displays both the pressure calculated by the calculation unit 23 and the proper pressure read from the memory card 90, on the digital display device 70. Moreover, in the case where the state in which the pressure calculated by the calculation unit 23 exceeds the proper pressure continues for the fixed period (e.g. 10 seconds), the display control unit 25 performs warning lamp (warning information) flashing display on the warning display device 71.

The sound control unit 26 in the information processing device 20 outputs a predetermined warning sound from the sound output unit 80, in the case where the state in which the pressure calculated by the calculation unit 23 exceeds the proper pressure continues for the fixed period (e.g. 10 seconds). Moreover, in the case where the number of operations counted by the operation number counting unit 22 exceeds a predetermined number corresponding to an effective period (e.g. 3 months) of the predetermined proper pressure, the sound control unit 26 in this embodiment outputs a predetermined warning sound (e.g. sound "Please receive guidance on proper pressure from trainer.") from the sound output device 80. Furthermore, in the case where the number of operations counted by the operation number counting unit 22 exceeds a predetermined number corresponding to a durable period (e.g. 5 years) of the muscle building tool 1, the sound control unit 26 outputs a predetermined warning sound (e.g. sound "Thank you for using our product for long time. Please replace it with new tool.") from the sound output device 80.

The function stop unit 27 in the information processing device 20 stops all functions of the information processing device 20 in the case where the number of operations counted by the operation number counting unit 22 exceeds the predetermined number corresponding to the durable period (e.g. 5 years) of the muscle building tool 1. The user can therefore be prevented from mistakenly using the muscle building tool 1 whose durable period has elapsed.

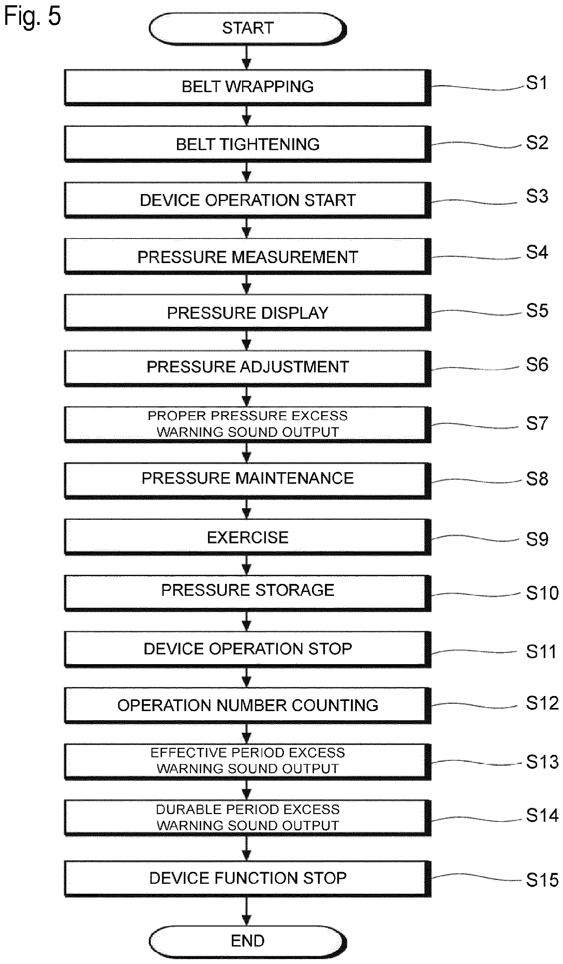

The following describes a use method of the muscle building tool 1 according to this embodiment, with reference to a flowchart in FIG. 5. Note that the use method includes a control method of the muscle building tool 1.

First, the user wraps the belt 10 of the muscle building tool 1 around a part, near the heart, of at least one (e.g. the upper left arm including biceps) of the four limbs including a muscle (or muscles) which the user wants to build, and forms a loop shape (belt wrapping step: S1). When doing so, the user folds the belt 10 back by passing the other end 10b of the belt 10 through the buckle 50 attached to the end 10a of the belt 10. It is assumed that, at this time, the memory card 90 to which the proper pressure is input by the trainer beforehand has been installed in the memory installation unit 91 of the information processing device 20 in the muscle building tool 1.

Next, the user tightens the belt 10 while holding the other end 10b of the belt 10 passed through the buckle 50 (belt tightening step: S2). When the tension acting on the belt 10 exceeds the predetermined threshold (e.g. 15 mmHg) as a result of the tightening, the information processing device 20 in the muscle building tool 1 automatically starts the operation (operation start step: S3). The calculation unit 23 in the information processing device 20 which has started the operation calculates the pressure applied to the muscle based on the electrical signal output from each pressure sensitive element 30 (pressure measurement step: S4). The display control unit 25 in the information processing device 20 displays both the pressure (actual pressure) calculated by the calculation unit 23 and the proper pressure read from the memory card 90, on the digital display device 70 (information display step: S5).

Following this, the user checks whether or not the actual pressure matches the proper pressure, by viewing the digital display device 70. In the case where the actual pressure does not match the proper pressure, the user adjusts a tightening force of the belt 10 while holding the other end 10b of the belt 10 passed through the buckle 50, to match the actual pressure to the proper pressure (pressure adjustment step: S6). Even if the tension acting on the belt 10 falls below or equal to the predetermined threshold during the adjustment, the counting correction function of the operation number counting unit 22 prevents this operation from being counted.

In the case where, during the adjustment of the tightening force of the belt 10, the actual pressure exceeds the proper pressure and this state in which the actual pressure exceeds the proper pressure continues for the fixed period (e.g. 10 seconds), the warning lamp flashes on the warning display device 71 in the information processing device 20, and the predetermined warning sound is output from the sound output unit 80 (proper pressure excess warning sound output step: S7). This enables the user to know that the state in which the actual pressure exceeds the proper pressure continues for the fixed period.

After checking that the actual pressure matches the proper pressure, the user maintains the loop shape of the belt 10 using the hook-and-loop fastener 40. Thus, the actual pressure equal to the proper pressure is applied to the desired muscle (pressure maintenance step: S8). The user then performs light exercise, while maintaining the loop shape of the belt 10 (while maintaining the constant actual pressure applied to the muscle by the belt 10). By maintaining the constant actual pressure in this way, it is possible to restrict blood flow in the muscle properly at a constant level, as a result of which the muscle can be built safely (exercise step: S9).

During the exercise, the maximum value of the actual pressure is written to the memory card 90. In the case where the state in which the actual pressure exceeds the proper pressure continues for the fixed period (e.g. 10 seconds) due to some factor, the information that the state in which the actual pressure exceeds the proper pressure continues for the fixed period is written to the memory card 90 (information storage step: S10). After the exercise, when the user unfastens the hook-and-loop fastener 40 to reduce the tightening force of the belt 10 and as a result the tension acting on the belt 10 falls below or equal to the predetermined threshold, the operation of the information processing device 20 is automatically stopped (operation stop step: S11).

The operation of the information processing device 20 is counted by the operation number counting unit 22 in the case where the exercise is performed using the muscle building tool 1 in the above-mentioned manner (operation number counting step: S12). Subsequently, the operation of the information processing device 20 is counted each time similar exercise is performed. In the case where the number of operations exceeds the predetermined number corresponding to the effective period (e.g. 3 months) of the predetermined proper pressure, the predetermined warning sound (e.g. sound "Please receive guidance on proper pressure from trainer.") is output from the sound output device 80 (effective period excess warning sound output step: S13). This enables the user to know that the effective period of the set proper pressure has elapsed, and set a new proper pressure.

Further, in the case where the number of operations exceeds the predetermined number corresponding to the durable period (e.g. 5 years) of the muscle building tool 1, the predetermined warning sound (e.g. sound "Thank you for using our product for long time. Please replace it with new tool.") is output from the sound output device 80 (durable period excess warning sound output step: S14). This enables the user to know that the durable period of the muscle building tool 1 has elapsed and it is time to purchase a new muscle building tool. Note that, in the case where the number of operations exceeds the predetermined number corresponding to the durable period (e.g. 5 years) of the muscle building tool 1, all functions of the information processing device 20 are stopped (function stop step: S15). The user can therefore be prevented from mistakenly using the muscle building tool 1 whose durable period has elapsed.

In the muscle building tool 1 according to the embodiment described above, in the case where the tension acting on the belt 10 when wrapping the belt 10 exceeds the predetermined threshold, the information processing device 20 can be automatically operated to measure the pressure. This eliminates the need to provide and manipulate an additional switch or the like for operating the information processing device 20, and enables the measurement of the pressure to be started immediately after the belt 10 is wrapped around, for example, the user's upper arm. Since the muscle building tool 1 does not need a pressure receiving bag or a tube unlike the conventional muscle building tool, the tool as a whole is compact and easy to use during training.

In the muscle building tool 1 according to the embodiment described above, the pressure sensitive element 30 is placed in the longitudinally substantially middle area of the belt 10 (position away from the ends 10a and 10b of the belt 10). This ensures that, for example when the belt 10 is wrapped around the user's upper arm and the pressure is applied while holding the end 10b of the belt 10, the pressure sensitive element 30 at the position away from the ends 10a and 10b of the belt 10 is closely attached to the upper arm. Hence, a situation where the pressure cannot be accurately measured due to a gap formed between the pressure sensitive element 30 and the human body can be avoided, which contributes to improved pressure measurement accuracy.

In the muscle building tool 1 according to the embodiment described above, it is possible to arrange the plurality of pressure sensitive elements 30 and calculate the mean value of the pressure based on the plurality of electrical signals output from the plurality of pressure sensitive elements 30. This reduces the possibility of any measurement error as compared with the case of measuring the pressure using a single pressure sensitive element, so that the pressure measurement accuracy can be further improved.

In the muscle building tool 1 according to the embodiment described above, in the case where the tension acting on the belt 10 falls below or equal to the predetermined threshold, the operation of the information processing device 20 can be automatically stopped. This eliminates the need to provide and manipulate an additional switch or the like for stopping the information processing device 20, and enables the measurement of the pressure to be stopped immediately after the belt 10 is removed. In addition, this automatic stop function can prevent wasting power.

In the muscle building tool 1 according to the embodiment described above, the maximum value of the pressure (actual pressure) calculated by the calculation unit 23 in the information processing device 20 can be stored in the memory card 90. Therefore, the maximum value of the actual pressure during the operation of the information processing device 20 can be confirmed subsequently.

In the muscle building tool 1 according to the embodiment described above, in the case where the state in which the actual pressure exceeds the proper pressure continues for the fixed period, the information that the state in which the actual pressure exceeds the proper pressure continues for the fixed period can be stored in the memory card 90. This enables the user to subsequently confirm the fact that the actual pressure exceeding the proper pressure is continuously applied for the fixed period.

In the muscle building tool 1 according to the embodiment described above, the display device 70 is placed near the buckle 50, so that the user can easily view information displayed on the display device 70. For example, when the belt 10 is wrapped around the user's upper arm and the pressure is applied to the muscle while folding the belt 10 back by passing, through the buckle 50 attached to the end 10a of the belt 10, the other end 10b of the belt 10, the user can easily view information such as the pressure because the display device 70 is placed near the buckle 50 (placed at the position where the viewing by the user is easiest).

In the muscle building tool 1 according to the embodiment described above, both the actual pressure and the proper pressure can be displayed on the display device 70. This enables the user to easily compare the actual pressure and the proper pressure, and adjust the pressure so that the actual pressure matches the proper pressure.

In the muscle building tool 1 according to the embodiment described above, in the case where the state in which the actual pressure exceeds the proper pressure continues for the fixed period, the predetermined warning information can be displayed on the warning display device 71 to inform the user.

In the muscle building tool 1 according to the embodiment described above, in the case where the state in which the actual pressure exceeds the proper pressure continues for the fixed period, the predetermined warning sound can be output from the sound output device 80 to inform the user.

In the muscle building tool 1 according to the embodiment described above, in the case where the number of uses of the muscle building tool 1 (the number of operations of the information processing device 20) exceeds the predetermined number, the predetermined warning sound can be output from the sound output device 80 to inform the user on the assumption that the effective period of the proper pressure set on the user basis has elapsed. This enables the user to know that the effective period of the set proper pressure has elapsed, and set a new proper pressure.

In the muscle building tool 1 according to the embodiment described above, in the case where the number of uses of the muscle building tool 1 (the number of operations of the information processing device 20) exceeds the predetermined number, the predetermined warning sound can be output from the sound output device 80 to inform the user on the assumption that the durable period of the muscle building tool 1 has elapsed. This enables the user to know that the durable period of the muscle building tool 1 has elapsed and it is time to purchase a new muscle building tool.

In the muscle building tool 1 according to the embodiment described above, in the case where the number of uses of the muscle building tool 1 (the number of operations of the information processing device 20) exceeds the predetermined number, the function of the information processing device 20 can be stopped on the assumption that the durable period of the muscle building tool 1 has elapsed. The user can therefore be prevented from mistakenly using the muscle building tool 1 whose durable period has elapsed.

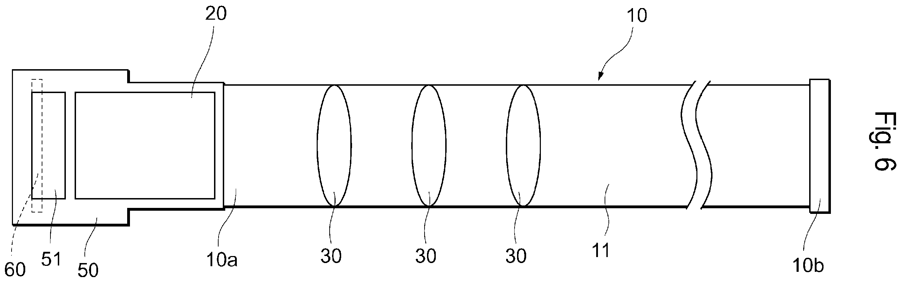

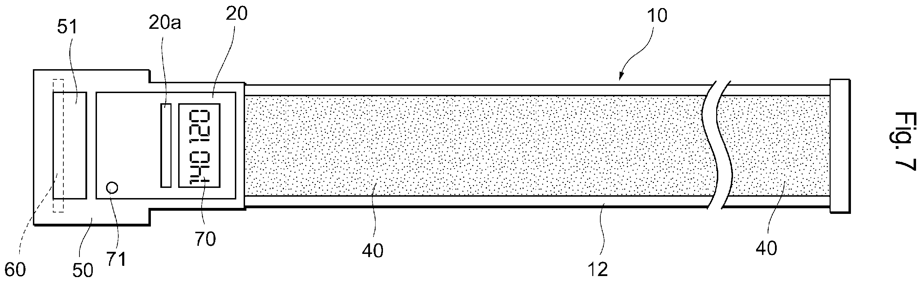

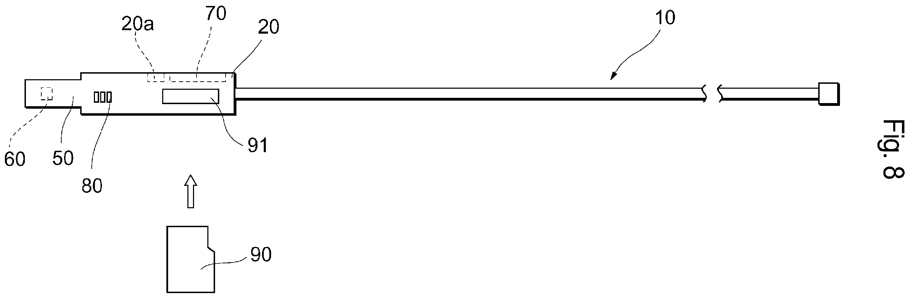

Though the example of placing the tension sensor 60 at the connecting portion between the information processing device 20 and the buckle 50 is shown in the embodiment described above, the position of the tension sensor 60 is not limited to this. For instance, the tension sensor 60 may be placed at a frame portion 51 that forms a hole of the buckle 50, as shown in FIGS. 6 to 8. Moreover, though the example of driving the information processing device 20 with power generated by the solar battery 20a is shown in the embodiment described above, a button battery or the like may be used instead of the solar battery 20a.

Though the example where the storage control unit 24 writes the maximum value of the pressure calculated by the calculation unit 23 during the operation of the information processing device 20 to the memory card 90 is shown in the embodiment described above, the pressure calculated by the calculation unit 23 may be written to the memory card 90 in association with elapsed time from the start of the operation of the information processing device 20. As a result, the time history of the actual pressure can be confirmed subsequently. Alternatively, each time a given period (e.g. 5 minutes) has elapsed from the start of the operation of the information processing device 20, the maximum value of the pressure calculated by the calculation unit 23 in the given period (5 minutes) may be written to the memory card 90. As a result, the maximum value of the actual pressure per the given period from the start of the operation of the information processing device 20 can be confirmed subsequently.

Though the example of writing the pressure calculated by the calculation unit 23 in the information processing device 20 to the memory card 90 to manage the pressure is shown in the embodiment described above, the pressure calculated by the calculation unit 23 may be sent to an external management device via wireless communication means and managed in the management device.

The present invention is not limited to the embodiment described above. Any design change appropriately made by a person skilled in the art to the embodiment is also included in the scope of the present invention, as long as it contains the features of the present invention. That is, the components in the embodiment as well as the positions, materials, conditions, shapes, sizes, and the like of the components are not limited to those illustrated above, and may be changed as required. Moreover, the components in the embodiment may be combined as long as it is technically possible, and such combination is also included in the scope of the present invention as long as it contains the features of the present invention as defined in the appended claims.

## Claims

1. A muscle building tool used to build a muscle of a limb by applying a predetermined pressure to at least one of a user's four limbs to restrict blood flow in the muscle of the limb, the muscle building tool comprising:
a belt (10) that is wrapped around the at least one of the four limbs and applies the pressure to the muscle; and
a shape maintenance member (40) that maintains a loop shape of the belt in a state of applying the pressure to the muscle; **characterized in that** the muscle building tool further comprises:
a tension measurement device (60) that measures a tension acting on the belt; and
a pressure measurement device connected to the tension measurement device (60), wherein the pressure measurement device is configured to automatically start an operation when the tension measured by the tension measurement device (60) exceeds a predetermined threshold, and measures the pressure applied to the muscle.

2. The muscle building tool according to claim 1,
wherein the pressure measurement device includes: a pressure sensitive element (30) that is placed in a longitudinally substantially middle area of a surface of the belt facing the muscle, and outputs an electrical signal corresponding to the pressure applied to the muscle; and a calculation unit (20) that calculates the pressure based on the electrical signal output from the pressure sensitive element.

3. The muscle building tool according to claim 2,
wherein a plurality of pressure sensitive elements (30) are arranged at predetermined intervals in the longitudinally substantially middle area, and
wherein the calculation unit (23) calculates a mean value of the pressure based on a plurality of electrical signals output from the plurality of pressure sensitive elements.

4. The muscle building tool according to claim 2 or 3,
wherein the pressure measurement device is configured to automatically stop the operation when, after the pressure measurement device is activated, the tension measured by the tension measurement device (60) falls below or equal to the predetermined threshold.

5. The muscle building tool according to any one of claims 2 to 4, comprising:
an information storage medium (90) configured to store various information; and
a storage control unit (24) that stores the pressure calculated by the calculation unit in the pressure measurement device, in the information storage medium (90).

6. The muscle building tool according to claim 5,
wherein the storage control unit (24) stores a maximum value of the pressure calculated by the calculation unit (23) during the operation of the pressure measurement device, in the information storage medium (90).

7. The muscle building tool according to claim 5 or 6,
wherein, in the case where the pressure calculated by the calculation unit (23) in the pressure measurement device exceeds a predetermined proper pressure and a state in which the pressure calculated by the calculation unit (23) exceeds the proper pressure continues for a fixed period, the storage control unit (24) stores, in the information storage medium (90), information that the state in which the pressure calculated by the calculation unit (23) exceeds the proper pressure continues for the fixed period.

8. The muscle building tool according to any one of claims 2 to 7, comprising:
a display device (70) for displaying various information; and
a display control unit (25) that displays various information including the pressure calculated by the calculation unit (23) in the pressure measurement device, on the display device (70).

9. The muscle building tool according to claim 8,
wherein a folding portion is provided at one end of the belt, the folding portion being used to fold the belt back by passing the other end of the belt through the folding portion when wrapping the belt, and wherein the display device (70) is placed near the folding portion.

10. The muscle building tool according to claim 8 or 9,
wherein the display control unit (25) displays both the pressure calculated by the calculation unit in the pressure measurement device and a predetermined proper pressure, on the display device (70).

11. The muscle building tool according to claim 10, wherein the display control unit (25) displays predetermined warning information on the display device (70), in the case where the pressure calculated by the calculation unit (23) in the pressure measurement device exceeds the proper pressure and a state in which the pressure calculated by the calculation unit (23) exceeds the proper pressure continues for a fixed period.

12. The muscle building tool according to any one of claims 2 to 11, comprising:
a sound output device (80) for outputting a sound; and
a sound control unit (26) that outputs a predetermined warning sound from the sound output device, in the case where the pressure calculated by the calculation unit (23) in the pressure measurement device exceeds a predetermined proper pressure and a state in which the pressure calculated by the calculation unit (23) exceeds the proper pressure continues for a fixed period.

13. The muscle building tool according to any one of claims 1 to 12,
wherein the pressure measurement device includes an operation number counting unit (22) that counts the number of operations of the pressure measurement device, and
wherein the muscle building tool comprises:
a sound output device (80) for outputting a sound; and
a sound control unit (26) that outputs a predetermined warning sound from the sound output device, in the case where the number of operations counted by the operation number counting unit (22) in the pressure measurement device exceeds a predetermined number corresponding to an effective period of a predetermined proper pressure.

14. The muscle building tool according to claim 13,
wherein the sound control unit (26) outputs a predetermined warning sound from the sound output device (80), in the case where the number of operations counted by the operation number counting unit (22) in the pressure measurement device exceeds a predetermined number corresponding to a durable period of the muscle building tool.

15. The muscle building tool according to claim 14,
wherein the pressure measurement device includes a function stop unit (27) that stops a function of the pressure measurement device, in the case where the number of operations counted by the operation number counting unit (22) exceeds the predetermined number corresponding to the durable period of the muscle building tool.

## Patentansprüche

1. Eine muskelaufbauende Vorrichtung, die zum Aufbau eines Muskels einer Gliedmaße verwendet wird, indem ein vorgegebener Druck auf mindestens eine der vier Gliedmaßen eines Benutzers ausgeübt wird, um den Blutfluss in den Muskel der Gliedmaße einzuschränken, wobei die muskelaufbauende Vorrichtung Folgendes umfasst:
einen Gurt (10), der um mindestens eine der vier Gliedmaßen herum angelegt wird und den Druck auf den Muskel ausübt; und
ein Formerhaltungselement (40), das in einem Zustand, in dem Druck auf den Muskel ausgeübt wird, eine Schlingenform des Gurts aufrecht erhält; **dadurch gekennzeichnet, dass** die muskelaufbauende Vorrichtung weiter Folgendes umfasst:
eine Spannungsmessvorrichtung (60), die eine auf den Gurt wirkende Spannung misst; und
eine mit der Spannungsmessvorrichtung (60) verbundene Druckmessvorrichtung, wobei die Druckmessvorrichtung so konfiguriert ist, dass sie automatisch einen Betriebsvorgang startet, wenn die von der Spannungsmessvorrichtung (60) gemessene Spannung einen vorgegebenen Grenzwert überschreitet, und den auf den Muskel ausgeübten Druck misst.

2. Die muskelaufbauende Vorrichtung nach Anspruch 1,
wobei die Druckmessvorrichtung Folgendes umfasst: ein druckempfindliches Element (30), das in einen longitudinal im Wesentlichen mittigen Bereich einer dem Muskel zugewandten Oberfläche des Gurts platziert wird und ein elektrisches Signal ausgibt, das dem auf den Muskel ausgeübten Druck entspricht; und eine Berechnungseinheit (20), die den Druck basierend auf dem von dem druckempfindlichen Element ausgegebenen elektrischen Signal berechnet.

3. Die muskelaufbauende Vorrichtung nach Anspruch 2,
wobei mehrere druckempfindliche Elemente (30) in vorgegebenen Abständen in dem longitudinal im Wesentlichen mittigen Bereich angeordnet sind, und
wobei die Berechnungseinheit (23) einen Mittelwert des Drucks basierend auf mehreren elektrischen Signalen berechnet, die von den mehreren druckempfindlichen Elementen ausgegeben werden.

4. Die muskelaufbauende Vorrichtung nach Anspruch 2 oder 3,
wobei die Druckmessvorrichtung so konfiguriert ist, dass sie den Betriebsvorgang stoppt, wenn nach Aktivierung der Druckmessvorrichtung die von der Spannungsmessvorrichtung (60) gemessene Spannung unter oder auf den vorgegebenen Grenzwert fällt.

5. Die muskelaufbauende Vorrichtung nach einem der Ansprüche 2 bis 4, umfassend:
ein Informationsspeichermedium (90), das für die Speicherung verschiedener Informationen konfiguriert ist; und
eine Speicherungssteuereinheit (24), die den von der Berechnungseinheit in der Druckmessvorrichtung berechneten Druck im Informationsspeichermedium (90) speichert.

6. Die muskelaufbauende Vorrichtung nach Anspruch 5,
wobei die Speicherungssteuereinheit (24) einen maximalen Druckwert, der von der Berechnungseinheit (23) während des Betriebsvorgangs der Druckmessvorrichtung gemessen berechnet wird, im Informationsspeichermedium (90) speichert.

7. Die muskelaufbauende Vorrichtung nach Anspruch 5 oder 6,
wobei, falls der von der Berechnungseinheit (23) in der Druckmessvorrichtung berechnete Druck einen vorgegebenen ordnungsgemäßen Druck übersteigt und der Zustand, in dem der von der Berechnungseinheit (23) berechnete Druck den ordnungsgemäßen Druck überschreitet, für einen festgelegten Zeitraum andauert, die Speicherungssteuereinheit (24) im Informationsspeichermedium (90) die Information speichert, dass der Zustand, in dem der von der Berechnungseinheit (23) berechnete Druck den ordnungsgemäßen Druck überschreitet, für den festgelegten Zeitraum andauert.

8. Die muskelaufbauende Vorrichtung nach einem der Ansprüche 2 bis 7, umfassend:
eine Anzeigevorrichtung (70) zur Anzeige verschiedener Informationen;
eine Anzeigesteuereinheit (25), die verschiedene Informationen, einschließlich des von der Berechnungseinheit (23) in der Druckmessvorrichtung berechneten Drucks, auf der Anzeigevorrichtung (70) anzeigt.

9. Die muskelaufbauende Vorrichtung nach Anspruch 8,
wobei an einem Ende des Gurts ein Faltabschnitt vorgesehen ist und der Faltabschnitt dazu verwendet wird, den Gurt zurückzufalten, indem beim Anlegen des Gurts das andere Ende des Gurts durch den Faltabschnitt geführt wird, und
wobei die Anzeigevorrichtung (70) in der Nähe des Faltabschnitts platziert ist.

10. Die muskelaufbauende Vorrichtung nach Anspruch 8 oder 9,
wobei die Anzeigesteuereinheit (25) sowohl den von der Berechnungseinheit in der Druckmessvorrichtung berechneten Druck als auch einen vorgegebenen ordnungsgemäßen Druck auf der Anzeigevorrichtung (70) anzeigt.

11. Die muskelaufbauende Vorrichtung nach Anspruch 10,
wobei die Anzeigesteuereinheit (25) eine vorgegebene Warninformation auf der Anzeigevorrichtung (70) anzeigt, falls der von der Berechnungseinheit (23) in der Druckmessvorrichtung berechnete Druck den ordnungsgemäßen Druck überschreitet und ein Zustand, in dem der von der Berechnungseinheit (23) berechnete Druck den ordnungsgemäßen Druck überschreitet, für einen festgelegten Zeitraum andauert.

12. Die muskelaufbauende Vorrichtung nach einem der Ansprüche 2 bis 11, umfassend:
eine Tonausgabevorrichtung (80) zur Ausgabe eines Tons;
eine Tonsteuereinheit (26), die einen vorgegebenen Warnton an der Tonausgabevorrichtung ausgibt, falls der von der Berechnungseinheit (23) in der Druckmessvorrichtung berechnete Druck einen vorgegebenen ordnungsgemäßen Druck überschreitet und ein Zustand, in dem der von der Berechnungseinheit (23) berechnete Druck den ordnungsgemäßen Druck überschreitet, für einen festgelegten Zeitraum andauert.

13. Die muskelaufbauende Vorrichtung nach einem der Ansprüche 1 bis 12,
wobei die Druckmessvorrichtung eine Einheit zur Zählung von Betriebsvorgängen (22) umfasst, die die Zahl der Betriebsvorgänge der Druckmessvorrichtung zählt, und
wobei die muskelaufbauende Vorrichtung Folgendes umfasst:
eine Tonausgabevorrichtung (80) zur Ausgabe eines Tons; und
eine Tonsteuereinheit (26), die einen vorgegebenen Warnton an der Tonausgabevorrichtung ausgibt, falls die Zahl der von der Einheit zur Zählung von Betriebsvorgängen (22) in der Druckmessvorrichtung gezählten Betriebsvorgänge eine vorgegebene Zahl überschreitet, die einem effektiven Zeitraum mit einem vorgegebenen ordnungsgemäßen Druck entspricht.

14. Die muskelaufbauende Vorrichtung nach Anspruch 13,
wobei die Tonsteuereinheit (26) einen vorgegebenen Warnton an der Tonausgabevorrichtung (80) ausgibt, falls die Zahl der von der Einheit zur Zählung von Betriebsvorgängen (22) in der Druckmessvorrichtung gezählten Betriebsvorgänge eine vorgegebene Zahl überschreitet, die einem andauernden Zeitraum der muskelaufbauenden Vorrichtung entspricht.

15. Die muskelaufbauende Vorrichtung nach Anspruch 14,
wobei die Druckmessvorrichtung eine Funktionsstoppeinheit (27) umfasst, die eine Funktion der Druckmessvorrichtung stoppt, falls die Zahl der von der Einheit zur Zählung von Betriebsvorgängen (22) gezählten Betriebsvorgänge die vorgegebene Zahl überschreitet, die dem andauernden Zeitraum der muskelaufbauenden Vorrichtung entspricht.

## Revendications

1. Instrument de développement musculaire utilisé pour développer un muscle d'un membre en appliquant une pression prédéfinie sur au moins un des quatre membres d'un utilisateur pour restreindre l'écoulement sanguin dans le muscle du membre, l'instrument de développement musculaire comportant :
une courroie (10) qui est enroulée autour dudit au moins un des quatre membres et applique la pression sur le muscle ; et
un élément de maintien de forme (40) qui maintient une forme de boucle de la courroie dans un état d'application de la pression sur le muscle ; **caractérisé en ce que** l'instrument de développement musculaire comporte en outré :
un dispositif de mesure de la tension (60) qui mesure une tension agissant sur la courroie ; et
un dispositif de mesure de la pression relié au dispositif de mesure de la tension (60), le dispositif de mesure de la pression étant configuré pour commencer automatiquement un actionnement lorsque la tension mesurée par le dispositif de mesure de la tension (60) dépasse un seuil prédéfini, et qui mesure la pression appliquée sur le muscle.

2. Instrument de développement musculaire selon la revendication 1,
dans lequel le dispositif de mesure de la pression comprend : un élément sensible à la pression (30) qui est placé dans une zone sensiblement médiane dans le sens longitudinal d'une surface de la courroie tournée vers le muscle, et délivre en sortie un signal électrique correspondant à la pression appliquée sur le muscle ; et une unité de calcul (20) qui calcule la pression sur la base de la sortie du signal électrique de l'élément sensible à la pression.

3. Instrument de développement musculaire selon la revendication 2,
dans lequel plusieurs éléments sensibles à la pression (30) sont agencés à des intervalles prédéfinis dans la zone sensiblement médiane dans le sens longitudinal, et
dans lequel l'unité de calcul (23) calcule une valeur moyenne de la pression sur la base de plusieurs sorties de signaux électriques en provenance desdits plusieurs éléments sensibles à la pression.

4. Instrument de développement musculaire selon la revendication 2 ou 3,
dans lequel le dispositif de mesure de la pression est configuré pour arrêter automatiquement l'actionnement lorsque, après activation du dispositif de mesure de la pression, la tension mesurée par le dispositif de mesure de la tension (60) tombe en dessous du seuil prédéfini ou est égale à celui-ci.

5. Instrument de développement musculaire selon l'une quelconque des revendications 2 à 4, comportant :
un support de mémorisation d'informations (90) configuré pour mémoriser diverses informations ; et
une unité de commande de mémorisation (24) qui mémorise la pression calculée par l'unité de calcul dans le dispositif de mesure de la pression, dans le support de mémorisation d'informations (90).

6. Instrument de développement musculaire selon la revendication 5,
dans lequel l'unité de commande de mémorisation (24) mémorise une valeur maximale de la pression calculée par l'unité de calcul (23) durant l'actionnement du dispositif de mesure de la pression, dans le support de mémorisation d'informations (90).

7. Instrument de développement musculaire selon la revendication 5 ou 6,
dans lequel, dans le cas où la pression calculée par l'unité de calcul (23) dans le dispositif de mesure de la pression dépasse une mesure convenable prédéfinie et un état dans lequel la pression calculée par l'unité de calcul (23) dépasse la pression convenable continue pendant une période fixe, l'unité de commande de mémorisation (24) mémorise, dans le support de mémorisation d'informations (90), les informations selon lesquelles l'état dans lequel la pression calculée par l'unité de calcul (23) dépasse la pression convenable continue pour la période fixe.

8. Instrument de développement musculaire selon l'une quelconque des revendications 2 à 7, comportant :
un dispositif d'affichage (70) permettant d'afficher diverses informations ; et
une unité de commande d'affichage (25) qui affiche diverses informations comprenant la pression calculée par l'unité de calcul (23) dans le dispositif de mesure de la pression, sur le dispositif d'affichage (70).

9. Instrument de développement musculaire selon la revendication 8,
dans lequel une partie pliante est fournie au niveau d'une extrémité de la courroie, la partie pliante étant utilisée pour replier la courroie en faisant passer l'autre extrémité de la courroie à travers la partie pliante lors de l'enroulement de la courroie, et
dans lequel le dispositif d'affichage (70) est placé près de la partie pliante.

10. Instrument de développement musculaire selon la revendication 8 ou 9,
dans lequel l'unité de commande d'affichage (25) affiche à la fois la pression calculée par l'unité de calcul dans le dispositif de mesure de la pression et une pression convenable prédéfinie, sur le dispositif d'affichage (70).

11. Instrument de développement musculaire selon la revendication 10,
dans lequel l'unité de commande d'affichage (25) affiche des informations d'avertissement prédéfinies sur le dispositif d'affichage (70), dans le cas où la pression calculée par l'unité de calcul (23) dans le dispositif de mesure de la pression dépasse la pression convenable et un état dans lequel la pression calculée par l'unité de calcul (23) dépasse la pression convenable continue pendant une période fixe.

12. Instrument de développement musculaire selon l'une quelconque des revendications 2 à 11, comportant
un dispositif de sortie de son (80) permettant de délivrer en sortie un son ; et
une unité de commande de son (26) qui délivre en sortie un son d'avertissement prédéfini en provenance du dispositif de sortie de son, dans le cas où la pression calculée par l'unité de calcul (23) dans le dispositif de mesure de la pression dépasse une pression convenable prédéfinie et un état dans lequel la pression calculée par l'unité de commande (23) dépasse la pression convenable continue pendant une période fixe.

13. Instrument de développement musculaire selon l'une quelconque des revendications 1 à 12,
dans lequel le dispositif de mesure de la pression comprend une unité de comptage du nombre d'actionnements (22) qui compte le nombre d'actionnements du dispositif de mesure de la pression, et
dans lequel l'instrument de développement musculaire comporte :
un dispositif de sortie de son (80) permettant de délivrer en sortie un son ; et
une unité de commande de son (26) qui délivre en sortie un son d'avertissement prédéfini en provenance du dispositif de sortie de son, dans le cas où le nombre d'actionnements comptés par l'unité de comptage du nombre d'actionnements (22) dans le dispositif de mesure de la pression, dépasse un nombre prédéfini correspondant à une période effective d'une pression convenable prédéfinie.

14. Instrument de développement musculaire selon la revendication 13,
dans lequel l'unité de commande de son (26) délivre en sortie un son d'avertissement prédéfini en provenance du dispositif de sortie de son (80), dans le cas où le nombre d'actionnements comptés par l'unité de comptage du nombre d'actionnements (22) dans le dispositif de mesure de la pression dépasse un nombre prédéfini correspondant à une période durable de l'instrument de développement musculaire.

15. Instrument de développement musculaire selon la revendication 14,
dans lequel le dispositif de mesure de la pression comprend une unité d'arrêt de fonction (27) qui arrête une fonction du dispositif de mesure de la pression, dans le cas où le nombre d'actionnements comptés par l'unité de comptage du nombre d'actionnements (22) dépasse le nombre prédéfini correspondant à la période durable de l'instrument de développement musculaire.
